Europäisches Patentamt

European Patent Office

Office européen des brevets

(19)

(11) Veröffentlichungsnummer: **0 111 626**
**B1**

# (12) EUROPÄISCHE PATENTSCHRIFT

(45) Veröffentlichungstag der Patentschrift:
23.09.87

(21) Anmeldenummer: 83107189.9

(22) Anmeldetag: 22.07.83

(51) Int. Cl.⁴: **C 07 D 303/42,** C 08 G 63/66,
C 08 G 63/48

(54) **Verfahren zur Herstellung modifizierter Triglyceride mit im Mittel Epoxid- u. Hydroxyl- sowie Ethergruppen.**

(30) Priorität: 16.12.82 DE 3246612

(43) Veröffentlichungstag der Anmeldung:
27.06.84 Patentblatt 84/26

(45) Bekanntmachung des Hinweises auf die Patenterteilung:
23.09.87 Patentblatt 87/39

(84) Benannte Vertragsstaaten:
AT BE CH DE FR GB IT LI NL SE

(56) Entgegenhaltungen:
DD-A-132 018
FR-A-1 135 898
US-A-3 070 608

Die Akte enthält technische Angaben, die nach
dem Eingang der Anmeldung eingereicht wurden
und die nicht in dieser Patentschrift enthalten sind.
(73) Patentinhaber: Henkel Kommanditgesellschaft auf
Aktien, Postfach 1100 Henkelstrasse 67, D-4000
Düsseldorf- Holthausen (DE)

(72) Erfinder: Meffert, Alfred, Dr., Marie- Curie-
Strasse 10, D-4019 Monheim (DE)
Erfinder: Kluth, Hermann, Degerstrasse 48, D-4000
Düsseldorf 1 (DE)

## Beschreibung

Die Erfindung liegt auf dem Gebiet organischer Produkte, die sich von nachwachsenden Rohstoffen ableiten. Sie betrifft ein Verfahren zur Herstellung modifizierter Triglyceride, die als reaktive Gruppen sowohl Epoxidgruppen als auch Hydroxylgruppen und Ethergruppen aufweisen. Die neuen modifizierten Triglyceride können zur Herstellung von Polymeren beispielsweise von Alkydharzen verwendet werden.

Es ist seit langem bekannt, natürlich vorkommende Öle und Fette (Triglyceride von Fettsäuren beziehungsweise Fettsäuregemischen, die wenigstens anteilsweise olefinisch ungesättigte Doppelbindungen enthalten) der Epoxidation zu unterwerfen und dabei eine oder sämtliche Doppelbindungen im Molekül in Epoxidringe zu überführen. Ein dazu geeignetes Verfahren wird z. B. von Thomas W. Findley et al. in J. Am. Chem. Soc. 67, 413 (1945) beschrieben.

Es ist auch bekannt, epoxidierte Triglyceride z. B. epoxidierten Rindertalg durch Ringöffnung mit mehrfunktionellen Alkoholen wie z. B. Trimethylolpropan in höherfunktionelle Alkohole zu überführen. Ein entsprechendes Verfahren wird beispielsweise von A. Bilyk et al. im Journal of the American Oil Chemist Society 51, 119 (1974) beschrieben.

Ziel der bekannten Ringöffnungsverfahren ist es, sämtliche Epoxidgruppen des jeweils eingesetzten epoxierten Triglycerids zur Reaktion zu bringen. Es wird dabei in Kauf genommen, daß bei den angewendeten, meist recht harten Reaktionsbedingungen auch Umesterung auftritt.

Die Lehre der DD-A-132 018 beschreibt die Umsetzung von epoxidierten Triglyceriden mit Kondensaten aus Butandiol-(1,3) unter Öffnung der Epoxidringe. Zur Erhöhung der Lagerstabilität des Produktes wird vorgeschlagen, einen Restepoxidgehalt von ca. 1 % aufrechtzuerhalten.

Im Zuge der Bemühungen, petrochemische Produkte durch nachwachsende Rohstoffe zu ersetzen, bestand insbesondere für das Anwendungsgebiet Alkydharze der Bedarf nach einem Rohstoff, welcher sowohl Epoxidgruppen als auch sekundäre Hydroxylgruppen sowie Ethergruppen aufweist und zumindest überwiegend oleochemischer Natur ist.

Aufgabe der Erfindung ist es somit, ein Verfahren zur Ringöffnung von epoxidierten Triglyceriden mit Alkoholen bereitzustellen, das es erlaubt, modifizierte Triglyceride mit Epoxidgruppen und sekundären Hydroxylgruppen sowie Ethergruppen (teilweise ringgeöffnete epoxydierte Triglyceride) herzustellen. Insbesondere ist es Aufgabe der Erfindung, ein Verfahren zur teilweisen Ringöffnung epoxidierter Triglyceride mit $C_1$ bis $C_3$-Alkoholen zu schaffen. Eine weitere Aufgabe der Erfindung besteht darin, modifizierte Triglyceride bereitzustellen, welche Epoxidgruppen und Hydroxylgruppen sowie Alkylethergruppen aufweisen und beispielsweise zur Herstellung von verbesserten Alkydharzen geeignet sind.

Die Durchführung der erfindungsgemäßen Aufgabe gelang durch Schaffung eines Verfahrens zur partiellen Ringöffnung epoxidierter Triglyceride eines wenigstens anteilsweise olefinisch ungesättigte Fettsäuren enthaltenden Fettsäuregemisches mit ein- oder mehrwertigen Alkoholen in Gegenwart von Katalysatoren zur Herstellung von Triglycerid-Reaktionsprodukten, die insbesondere für die Modifizierung von Alkydharzen geeignet sind, dadurch gekennzeichnet, daß man die Reaktion durch Zerstören und/oder Entfernen des Katalysators und/oder des Alkoholreaktanten nach einem Umsatz von 20 bis 80 mol-%, bezogen auf Epoxidgruppen, abbricht.

Nach einer ersten Ausführungsform des erfindungsgemäßen Verfahrens wird die Ringöffnungsreaktion der epoxidierten Triglyceride mit den Alkoholen nach Umsetzung von 20 bis 80 Mol-% der Epoxidgruppen dadurch abgebrochen, daß der Katalysator zerstört wird. Bevorzugt katalysiert man mit Säuren, insbesondere mit Mineralsäuren wie Schwefelsäure, Phosphorsäure, Salzsäure, oder aber auch mit organischen Säuren wie Sulfonsäuren, beispielsweise p-Toluolsulfonsäure. Die katalytische Wirkung der Säuren wird durch Neutralisation aufgehoben. Geeignete Neutralisationsmittel sind Alkalialkoholate, z. B. Natriummethylat. Im einzelnen wird so vorgegangen, daß das epoxidierte Triglycerid mit einem hohen Überschuß an Alkohol, also z. B. mit einem 2 bis 10 molaren Überschuß bezogen auf Epoxidgruppen, versetzt wird. Danach wird der Reaktionsansatz in Gegenwart des sauren Katalysators bei Reaktionstemperaturen zwischen 50 und 110°C, vorzugsweise 60 bis 80°C, belassen. Das Fortschreiten der Reaktion kann über die Epoxidzahl bestimmt werden, welche sich durch Säuretitration, Gaschromatographie oder spektroskopische Methoden kontrollieren läßt. Sobald die Epoxidzahl auf den gewünschten, vorausberechneten Wert gefallen ist, wird das Neutralisationsmittel zugefügt. Der Überschuß an Alkohol kann hiernach gewünschtenfalls destillativ entfernt werden.

Nach einer weiteren Ausführungsform der Erfindung werden heterogene Katalysatoren verwendet. Geeignet sind z. B. Ionenaustauscherharze, so z. B. stark saure Ionenaustauscher oder stark basische Ionenaustauscher. Die Ionenaustauscherharze werden nach Erreichen der gewünschten Restepoxidzahl durch Filtration vom Filtrationsgemisch abgetrennt.

Nach einer weiteren verfahrensgemäßen Variante der Erfindung wird anstelle des Katalysators ein Reaktionspartner - vorzugsweise der im allgemeinen leichter flüchtige Alkohol - entfernt. Nach dieser Verfahrensweise ist es bevorzugt, am Ende der gewünschten Reaktionszeit auf Raumtemperatur oder auch darunter abzukühlen und den Alkohol durch Destillation zu entfernen. Bei dieser Vorgehensweise verbleibt der Katalysator im Reaktionsgemisch und kann somit für spätere weitere Umsetzungen verwendet werden.

Ziel des erfindungsgemäßen Verfahrens ist es, modifizierte Triglyceride herzustellen, die außer Epoxidgruppen auch sekundäre Hydroxylgruppen sowie Alkylethergruppen aufweisen. Nach einer bevorzugten Ausführungsform wird angestrebt, das Triglyceridgerust zumindest im wesentlichen, also zu mehr als 50 %, vorzugsweise zu mehr als 70 %, und insbesondere zu mehr als 90 %, zu erhalten. Um Umesterung weitgehend zu vermeiden, wendet man vorzugsweise Reaktionstemperaturen zwischen 50 und 110° C, insbesondere zwischen 60 und 80° C, an. Werden zur Ringöffnung Alkohole eingesetzt, die unter 80° C sieden, so kann beim jeweiligen Siedepunkt dieser Alkohole gearbeitet werden.

Das erfindungsgemäße Verfahren wird bevorzugt mit Alkoholen der Funktionalität 1 - 4, insbesondere 1 - 3, durchgeführt, wobei kurzkettige Alkohole reaktiver als langkettige sind. So können in einer speziellen Ausführungsform monofunktionelle Alkohole mit bis zu 8 Kohlenstoffatomen verwendet werden. Bevorzugt sind kurzkettige monofunktionelle Alkohole, also solche mit bis zu 3 C-Atomen wie Methanol, Ethanol, Propanol, Isopropanol oder Allylalkohol. Geeignet sind weiter verzweigte Alkohole wie Isobutanol oder Neopentylalkohol oder cyclische Alkohole wie Hexanol oder Benzylalkohol.

In einer weiteren Ausführungsform der Erfindung werden zu der Ringöffnung bifunktionlle Alkohole eingesetzt. Es ist hier bevorzugt, in einem Überschuß von mehr als 2 mol vorzugsweise in einem Überschuß von 4 bis 8 mol bezogen auf die zu öffnenden Epoxidgruppen zu arbeiten. Dadurch wird verhindert, daß durch Reaktion beider Hydroxylgruppen des bifunktionellen Alkoholes mit je einem Molekül epoxidierten Triglycerids in wesentlichem Umfang höher molekulare Produkte entstehen. Geeignete bifunktionelle Alkohole sind insbesondere Ethylenglykol und Propylenglykol, aber auch die isomeren Butandiole, Pentandiole oder Hexandiole. Bei Einsatz dieser bifunktionellen Hydroxyverbindungen zur Ringöffnung entstehen modifizierte Triglyceride, welche außer Epoxidgruppen, EEthergruppen und sekundären Hydroxylgruppen auch primäre Hydroxylgruppen enthalten. Dies ist aufgrund der höheren Reaktivität primärer Hydroxylgruppen für viele Anwendungen, z. B. für die Herstellung von Alkydharzen gewünscht.

Für manche Anwendungen kann eine weitere Ethergruppe im Molekül vorteilhaft sein. Um dieses zu erreichen, wird die Ringöffnung mit den Monoethern bifunktioneller Alkohole durchgeführt. Geeignete Monoether bifunktioneller Alkohole enthalten beispielsweise insgessamt 3 bis 8 Kohlenstoffatome, vorzugsweise 3 bis 5 Kohlenstoffatome. So sind z.

B. Ethylenglykolmonomethylether, -ethylether, -propylether, aber auch Diethylenglykol oder Ether des Diethylenglykols sowie die entsprechenden Verbindungen, die sich vom Propylenglykol ableiten, geeignet.

Eine weitere Ausführungsform der Erfindung betrifft schließlich die teilweise Ringöffnung epoxidierter Triglyceride durch trifunktionelle Alkohole mit 3 bis 6 C-Atomen. Bevorzugt sind hier vor allem Glycerin oder Trimethylolpropan. Da diese Alkohole relativ schwer flüchtig sind, werden sie nach Beendigung der Umsetzung im Hochvakuum, so z. B. in einem Dünnschichtverdampfer entfernt. Durch die Verwendung trifunktioneller Alkohole zur Ringöffnung entstehen modifizierte Triglyceride, die neben Epoxidgruppen und Ethergruppen zahlreiche Hydroxylgruppen aufweisen. Die damit verbundene höhere Hydrophilie ist für viele Anwendungen gewünscht.

Das erfindungsgemäße Verfahren kann mit einer Vielzahl epoxidierter Triglyceride pflanzlichen oder tierischen Ursprungs durchgeführt werden. Voraussetzung ist lediglich, daß ein substantieller Anteil an Epoxidgruppen vorhanden ist. So sind z. B. epoxidierte Triglyceride geeignet, die 2 bis 10 Gew.-% Epoxidsauerstoff aufweisen. Bevorzugt geeignet sind Produkte mit 4 bis 8,5 Gew.-% Epoxidsauerstoff. Sie sind herstellbar aus den folgenden Fetten, Ölen (geordnet nach steigender Ausgangsjodzahl): Rindertalg, Palmöl, Schmalz, Rizinusöl, Erdnußöl, Rüböl, sowie bevorzugt Baumwollsaatöl, Sojabohnenöl, Tranöl, Sonnenblumenöl, Leinöl. Besonders bevorzugte Rohstoffe sind epoxidiertes Sojabohnenöl mit Epoxidzahl 5,8 bis 6,5, epoxidiertes Sonnenblumenöl mit Epoxidzahl 5,6 bis 6,6, epoxidiertes Leinöl mit Epoxidzahl 8,2 bis 8,6, sowie epoxidiertes Tranöl mit Epoxidzahl 6,3 bis 6,7.

Nach dem erfindungsgemäßen Verfahren lassen sich modifizierte Triglyceride herstellen, welche einen hohen Restgehalt an Epoxidgruppen (etwa 80 %) und einen geringen Gehalt an Hydroxyl- und Alkylethergruppen (20 %) aufweisen. Es sind jedoch auch Produkte zugänglich, die einen geringen Gehalt an Restepoxidgruppen (20 %) und einen hohen Gehalt an Hydroxyl- und Alkylethergruppen (80 %) aufweisen, sowie alle Zwischenwerte. Für viele Anwendungen ist es gewünscht, daß die Anzahl an Epoxidgruppen und an Hydroxyl- und an Alkylethergruppen etwa ausgeglichen ist. Es ist daher bevorzugt, 30 bis 70 Mol-% der Epoxidgruppen der Ringöffnung zu unterwerfen, insbesondere jedoch 40 bis 60 mol-% der Epoxidgruppen.

Die erfindungsgemäßen Produkte eignen sich als Rohstoffe zur Herstellung von Polymeren, insbesondere von Alkydharzen. Diese können in Alkydharze sowohl über Epoxidgruppen als auch über Hydroxylgruppen einkondensiert werden und stellen somit insbesondere neue mehrfunktionelle Polykondensationsrohstoffe auf natürlicher Basis dar.

**Beispiele**

**Beispiel 1:**

1050 g epoxidiertes Sojaöl (6,1 Gew.-% Epoxidsauerstoff) wurden mit 385 g Methanol und 4 g konzentrierter Schwefelsäure auf Rückflußtemperatur erhitzt und 90 Minuten bei dieser Temperatur unter Rühren belassen. Anschließend wurden 7,3 g einer 30 %igen Natriummethylatlösung in Methanol zugegeben und überschüssiges Methanol zunächst bei Normaldruck dann im Vakuum entfernt. Es entstand ein modifiziertes Sojatriglycerid mit Epoxidgruppen, Hydroxyl- und Methylethergruppen. Die Restepoxidzahl betrug 3,26 Gew.-% Epoxidsauerstoff.

**Beispiel 2:**

250 g Ethylenglykol und 1,2 g konzentrierte Schwefelsäure wurden in einer Standardapparatur auf 90°C erhitzt. Es wurden 1050 g epoxidiertes Sojaöl (6,1 Gew.-% Epoxidsauerstoff) innerhalb von 20 Minuten zugegeben. Dabei wurde darauf geachtet, daß die Temperatur 100°C nicht überstieg. Nach 30 Minuten wurde die Reaktion durch Zugabe von 3,8 g einer 30 %igen Lösung von Natriummethylat in Methanol abgebrochen. Überschüssiges Ethylenglykol wurde im Vakuum (etwa $10^{-5}$ bar) abdestilliert. Das entstandene Produkt hatte einen Restepoxidgehalt von 2,5 Gew.-% Epoxidsauerstoff.

**Beispiel 3:**

In einer Standardreaktionsapparatur wurden 96 g Methanol und 0,5 g konzentrierte Schwefelsäure zum Sieden erhitzt. Es wurden dann 125 g epoxidiertes Sonnenblumenöl (6,4 Gew.-% Epoxidsauerstoff) innerhalb von 30 Minuten zugegeben und 4 Stunden unter Rückfluß des siedenden Methanols erhitzt. Anschließend wurden 0,7 g einer 30 %igen Natriummethylatlösung in Methanol zugegeben und überschüssiges Methanol abdestilliert. Das entstandene Produkt hatte 3,6 Gew.-% Epoxidsauerstoff.

**Beispiel 4:**

In einer Standardapparatur wurden 128 g Methanol und 2 g konzentrierte Schwefelsäure zum Sieden erhitzt und innerhalb von 25 Minuten mit 360 g epoxidiertem Leinöl (8,45 Gew.-% Epoxidsauerstoff) versetzt. Es wurde 30 Minuten unter Rückfluß des Methanols am Sieden gehalten, und anschließend wurde die Reaktion durch Zugabe von 3,1 g einer 30 Gew.-%igen Natriummethylatlösung in Methanol abgebrochen. Nach Abdestillieren des überschüssigen Methanols entstand ein Produkt mit 3,5 Gew.-% Epoxidsauerstoff.

**Beispiel 5:**

In einer Standardapparatur wurden 96 g Methanol und 0,5 g Schwefelsäure zum Sieden erhitzt und mit 123 g Tranölepoxid (6,5 Gew.-% Epoxidsauerstoff) versetzt. Man beließ den Reaktionsansatz 6 Stunden bei dieser Temperatur. Im Anschluß daran wurde der Katalysator mit 0,7 g einer 30 Gew.-%igen Lösung von Natriummethylat in Methanol neutralisiert. Nach Abdestillieren des überschüssigen Methanols entstand ein Produkt mit einem Restepoxidgehalt von 3,87 % Epoxidsauerstoff.

**Beispiel 6:**

Zur Bestimmung des Umesterungsgrades wurde Beispiel 1 wiederholt, jedoch wurde die Ringöffnung nicht abgebrochen sondern bis zum vollständigen Umsatz, der nach etwa 6 Stunden erreicht war, geführt. Die gaschromatographische Bestimmung ergab einen Gehalt von etwa 2 bis 3 Gew.-% an modifizierten Fettsäuremethylestern sowie weiteren 5 Gew.-% an flüchtigen Bestandteilen..

**Patentansprüche**

1. Verfahren zur partiellen Ringöffnung epoxidierter Triglyceride eines wenigstens anteilsweise olefinisch ungesättigte Fettsäuren enthaltenden Fettsäuregemisches mit ein- oder mehrwertigen Alkoholen in Gegenwart von Katalysatoren zur Herstellung von Triglycerid-Reaktionsprodukten unter Bewahrung eines Restepoxidgehalts, dadurch gekennzeichnet, daß man die Reaktion durch Zerstören und/oder Entfernen des Katalysators und/oder des Alkoholreaktanten nach einem Umsatz von 20 - 80 mol-%, bezogen auf Epoxidgruppen, abbricht.

2. Verfahren nach Anspruch 1, dadurch gekennzeichnet, daß zur Ringöffnung mindestens einer der folgenden Alkohole eingesetzt wird:

a) primäre oder sekundäre, gesättigte oder ungesättigte monofunktionelle Alkohole mit 1 - 8 C-Atomen, vorzugsweise 1 - 3 C-Atomen,

b) bifunktionelle Alkohole mit 2 - 6 C-Atomen, vorzugsweise 2 - 4 C-Atomen,

c) Monoether bifunktioneller Alkohole mit insgesamt 3 - 8 C-Atomen, vorzugsweise 3 - 5 C-Atomen,

d) trifunktionelle Alkohole mit 3 - 6 C-Atomen.

3. Verfahren nach den Ansprüchen 1 und 2, dadurch gekennzeichnet, daß zur Ringöffnung saure Katalysatoren eingesetzt werden, insbesondere Schwefelsäure, Phosphorsäure oder Sulfonsäuren, und daß diese nach Erreichen des gewünschten Umsatzes neutralisiert werden.

4. Verfahren nach den Ansprüchen 1 - 3, dadurch gekennzeichnet, daß man die Alkohole, bezogen auf vorhandene Epoxidgruppen im 2 - 10 molaren Überschuß einsetzt und gewünschtenfalls nach Neutralisation des Katalysators abdestilliert.

5. Verfahren nach den Ansprüchen 1 - 4, dadurch gekennzeichnet, daß die Ringöffnung ohne gleichzeitige substantielle Umesterung bewirkt wird und Temperaturen zwischen 50 und 110°C, vorzugsweise 60 und 80°C angewendet werden.

6. Verfahren nach den Ansprüchen 1 - 5, dadurch gekennzeichnet, daß man 30 - 70 mol-%, insbesondere 40- 60 mol-% der Epoxidgruppen öffnet.

7. Verfahren nach den Ansprüchen 1 - 6, dadurch gekennzeichnet, daß die eingesetzten epoxidierten Triglyceride 2 - 10, vorzugsweise 4 - 8,5 Gew.-% Epoxidsauerstoff enthalten.

8. Modifizierte Triglyceride mit Epoxidgruppen und Hydroxylgruppen sowie Alkylethergruppen, insbesondere für die Alkydharzsynthese, hergestellt nach einem der Verfahren 1 - 7.

9. Verwendung der modifizierten Triglyceride mit Epoxidgruppen und Hydroxylgruppen sowie Alkylethergruppen nach einem der Ansprüche 1 - 8 als Rohstoffe zur Herstellung von Polyestern, insbesondere von Alkydharzen.


## Claims

1. A process for the partial ring opening of epoxidized triglycerides of a fatty acid mixture containing at least partly olefinically unsaturated fatty acids with mono- or polyhydric alcohols in the presence of catalysts for the production of triglyceride reaction products retaining a residual epoxide content, characterized in that the reaction is terminated by destruction and/or removal of the catalyst and/or the alcohol reactant after a conversion of from 20 to 80 mole-%, based on epoxide groups.

2. A process as claimed in Claim 1, characterized in that at least one of the following alcohols is used for the ring opening reaction:
   a) primary or secondary, saturated or unsaturated, monohydric $C_1$-$C_8$ and preferably $C_1$-$C_3$ alcohols,
   b) dihydric $C_2$-$C_6$ and preferably $C_2$-$C_4$ alcohols,
   c) monoethers of dihydric alcohols containing a total of 3 to 8 and preferably 3 to 5 carbon atoms,
   d) trihydric $C_3$-$C_6$ alcohols.

3. A process as claimed in Claims 1 and 2, characterized in that acidic catalysts, more especially sulfuric acid, phosphoric acid or sulfonic acids, are used for the ring opening reaction and in that these acidic catalysts are neutralized after the desired conversion has been reached.

4. A process as claimed in Claims 1 to 3, characterized in that the alcohols are used in a 2 to 10 molar excess, based on the epoxide groups present, and if desired are distilled off after neutralization of the catalyst.

5. A process as claimed in Claims 1 to 4, characterized in that the ring opening reaction is carried out without substantial transesterification at temperatures of from 50 to 110° C and preferably at temperatures of from 60 to 80° C.

6. A process as claimed in Claims 1 to 5, characterized in that from 30 to 70 mole-% and more especially from 40 to 60 mole-% of the epoxide groups are opened.

7. A process as claimed in Claims 1 to 6, characterized in that the epoxidized triglycerides used contain from 2 to 10 % by weight and preferably from 4 to 8.5 % by weight epoxide oxygen.

8. Modified triglycerides containing epoxide groups and hydroxyl groups and also alkylether groups, more especially for the synthesis of alkyd resins, produced by the process claimed in Claims 1 to 7.

9. The use of the modified triglycerides containing epoxide groups and hydroxyl groups and also alkylether groups according to Claims 1 to 8 as raw materials for the production of polyesters, more especially alkyd resins.


## Revendications

1. Procédé pour l'ouverture partielle des noyaux de triglycérides époxydés d'un mélange d'acides gras contenant au moins en partie des acides gras oléfiniquement insaturés avec des alcools mono- ou polyvalents en présence de catalyseurs pour la fabrication de produits de réaction de triglycérides avec conservation d'une teneur résiduelle en époxy, caractérisé en ce qu'on interrompt la réaction par destruction et/ou élimination du catalyseur et/ou du réactif alcoolique après une conversion de 20 à 80 moles % par rapport aux groupes époxy.

2. Procédé selon la revendication 1, caractérisé en ce que pour l'ouverture des cycles on utilise au moins un des alcools suivants:
   a) des alcools monofonctionnels primaires ou secondaires, saturés ou non saturés, qui ont 1 à 8 atomes de carbone, de préférence 1 à 3 atomes de carbone,
   b) des alcools bifonctionnels ayant 2 à 6 atomes de carbone, de préférence 2 à 4 atomes de carbone,
   c) des monoéthers d'alcools bifonctionnels ayant en tout 3 à 8 atomes de carbone, de préférence 3 à 5 atomes de carbone,
   d) des alcools trifonctionnels ayant 3 à 6 atomes de carbone.

3. Procédé selon les revendications 1 et 2, caractérisé en ce que pour l'ouverture des cycles on utilise des catalyseurs acides, en particulier de l'acide sulfurique, de l'acide phosphorique ou des acides sulfoniques, et en ce qu'on neutralise ceux-ci après avoir atteint la conversion souhaitée.

4. Procédé selon les revendications 1 à 3, caractérisé en ce qu'on utilise les alcools en un excès 2 à 10 fois molaire par rapport aux groupes époxy présents et en ce qu'éventuellement après neutralisation du catalyseur on les sépare par distillation.

5. Procédé selon les revendications 1 à 4, caractérisé en ce qu'on réalise l'ouverture des cycles sans transestérification substantielle simultanée et en ce qu'on applique des températures entre 50 et 110° C, de préférence de 60 à 80° C.

6. Procédé selon les revendications 1 à 5, caractérisé en ce qu'on ouvre 30 à 70 moles %, en particulier 40 à 60 moles % des groupes époxy.

7. Procédé selon les revendications 1 à 6, caractérisé en ce que les triglycérides époxydés employés contiennent 2 à 10, de préférence 4 à 8,5 % en poids d'oxygène époxy.

8. Triglycérides modifiés avec des groupes époxy, des groupes hydroxyle de même que des groupes alcoyl-éther, en particulier pour la synthèse de résines alkydes, préparés selon l'un des procédés 1 à 7.

9. Utilisation des triglycérides modifiés avec des groupes époxy, des groupes hydroxyle de même que des groupes alcoyl-éther selon l'une des revendications 1 à 8 en tant que matières premières pour la fabrication de polyesters, en particulier de résines alkydes.